# DEMANDE DE BREVET EUROPEEN

(11) **EP 3 189 874 A1**
(43) Date de publication de la demande: **12.07.2017**
(21) Numéro de dépôt: 16290001.3
(22) Date de dépôt: 07.01.2016
(51) Int. Cl.: A61Q 19/10, A61K 8/92, A61K 8/02

(54) **CRÈME HYDRATANTE QUI SUPPRIME TOUTES LES TÂCHES DE PEINTURE À L'HUILE, DE PEINTURE LAQUÉES, LES TÂCHES DE FEUTRES, STYLOS ET CAMBOUIS SUR LA PEAU**

(71) Demandeur: Josyane, Tapiero, 75018 Paris (FR)
(72) Inventeur: Josyane, Tapiero, 75018 Paris (FR)

(57) **Abrégé**

L'invention concerne une crème hydratante qui contient uniquement des produits naturels, des agents hydratants et des huiles naturelles dont l'huile de monoï comme principal actif permettant ainsi de supprimer toutes les tâches de peintures, feutres, stylos, cambouis sans agresser la peau, sans risque d'allergies, non toxiques et nocifs pour la santé en laissant la peau douce et hydratée.

Cette crème comprend de l'huile de noix de coco pure, de l'huile d'olive vierge 1^{ère} pression, de l'huile d'amande douce, du beurre de karité jaune pur non traité et de l'huile de monoï.

## Description

La présente invention concerne une crème hydratante qui supprime toutes les tâches de peinture à l'huile, de peintures laquées, les tâches de feutres, stylos et cambouis sur la peau. Actuellement, l'ensemble des produits détachants sur le marché ne protègent pas la peau : en effet, ils sont composés d'agents et de produits nocifs pour la peau contenant généralement des alcools, du parabène, des colorants et produits chimiques. Ce problème peut être mis en évidence par le seul produit existant sur le marché tel que le White Spirit® qui est un produit très nocif pour la peau et la santé.
La crème selon l'invention permet de remédier à cet inconvénient. Elle comporte uniquement des produits naturels, des agents hydratants et des huiles naturelles qui protègent la peau ; cette crème ne contient aucun alcool, aucun produits chimiques, ni parabène et colorant.
La crème supprime toutes les tâches sur la peau, il suffit de l'étaler sur la peau et frotter. Cette crème laisse la peau douce et hydratée tout en la protégeant naturellement, sans laisser aucune traces visibles, ni de marques de rougeurs ou autres caractéristiques d'allergies.

La présente invention a donc pour premier objet une crème comprenant de l'huile de noix de coco pure, de l'huile d'olive vierge 1^{ère} pression, de l'huile d'amande douce, du beurre de karité jaune pur non traité et de l'huile de monoï.

Selon un aspect préféré, la crème est constituée de :
- Huile de noix de coco pure : 20 ml
- Huile d'olive vierge 1^{ère} pression : 55 ml
- Huile d'amande douce : 20 ml
- Beurre de karité jaune pur non traité : 50 grammes
- Huile de monoï : 20 inl

Un autre objet de l'invention concerne le procédé de préparation de la crème. Il consiste à mettre dans un récipient 50 grammes de beurre de karité jaune pur non traité, ensuite incorporer 20 ml d'huile d'amande douce et mélanger dans le sens des aiguilles d'une montre en écrasant le beurre de karité ; une fois le mélange homogène, y ajouter 20 ml d'huile de noix de coco pure et 20 ml d'huile de monoï de telle sorte que le mélange forme une crème homogène et enfin y ajouter 55 ml d'huile d'olive vierge 1^{ère} pression pour former la crème.

Les dessins annexés illustrent l'invention :

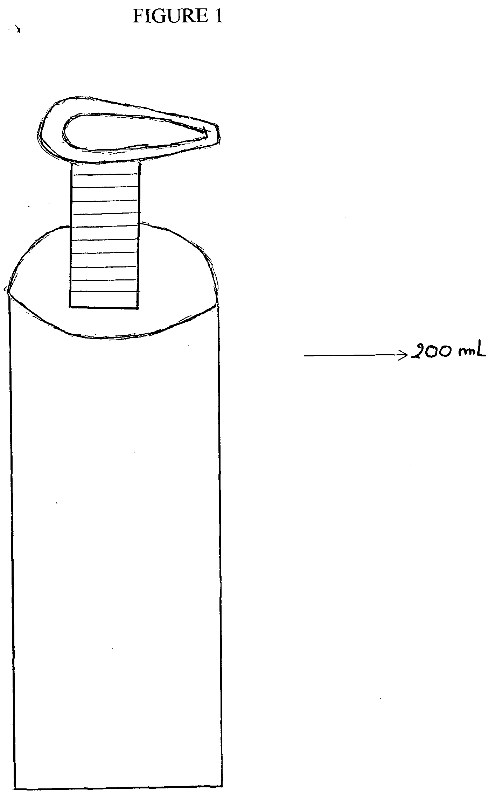

La figure 1 illustre la crème dans son flacon doseur de 200 ml.

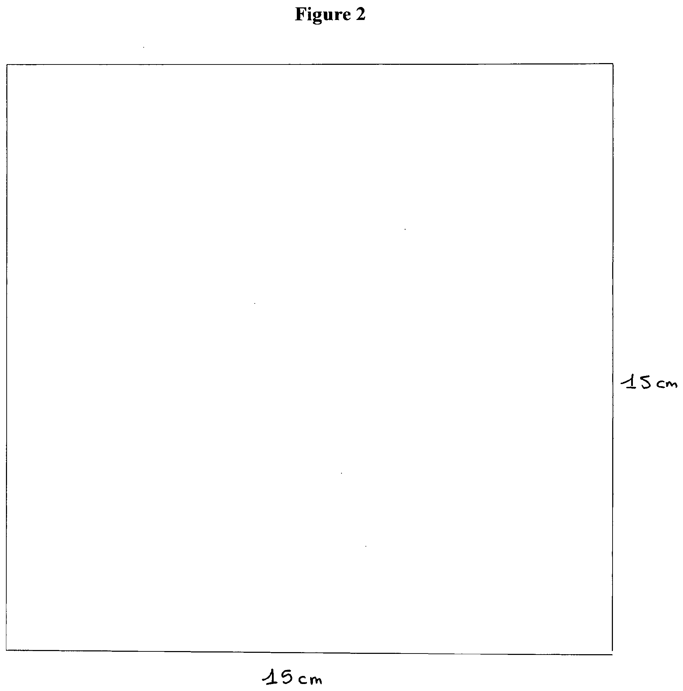

La figure 2 illustre la crème imprégnant une lingette nettoyante carrée de 15 cm de côté.
En référence à ces dessins, la crème peut être réalisée sous forme de crème à la texture onctueuse, introduite dans un flacon doseur de 200 ml en plastique avec une pression doseur, mais également dans des lingettes nettoyantes épaisses carrées de 15 cm de côté permettant une utilisation optimale, efficace et rapide que ce soit dans la rue, à la maison ou au travail. Les lingettes sont regroupées dans des paquets de 50.

Un dernier objet de la présente invention concerne l'utilisation de la crème pour supprimer toutes les tâches de peinture à l'huile, de peintures laquées, les tâches de feutres, stylos et cambouis sur la peau.

La crème selon l'invention est destinée à la vente dans la plupart des magasins de grandes surfaces, les surfaces de bricolage, les supermarchés, tout ce qui touche la grande distribution ; la vente s'effectuera aussi au niveau des parapharmacies touchant à la fois les professionnels et les particuliers dans leurs activités quotidiennes.

## Revendications

1. Crème nettoyante, hydrante, naturelle **caractérisée en ce qu'**elle est constituée d'une combinaison de plusieurs agents hydratants et d'huiles naturelles formant un complexe actif nettoyant et hydratant dont l'actif principal est l'huile de monoï : en effet, en plus de son action hydrante sur la peau: l'huile de monoï a un pouvoir nettoyant renforcé dans la combinaison avec les huiles présentes dans la crème, l'actif nettoyant de l'huile de monoï se révèle au fur et à mesure du mélange et de la combinaison des autres huiles ainsi que leur quantités, la formation du complexe hydratant et nettoyant forme la crème dont l'agent principal est l'huile de monoï, en l'absence d'huile de monoï , la crème n'a pas son pouvoir nettoyant et elle pert donc son activité inventive.
• Huile de noix de coco pure : 20 ml
• Huile d'olive vierge 1^{ère} pression : 55 ml
• Huile d'amande douce : 20 ml
• Beurre de karité jaune pur non traité : 50 grammes
• Huile de monoï : 20 ml

2. Procédé de préparation de la crème selon la revendication 1 **caractérisé en ce qu'**il consiste à mettre dans un récipient 50 grammes de beurre de karité jaune pur non traité, ensuite incorporer 20 ml d'huile d'amande douce et mélanger dans le sens des aiguilles d'une montre en écrasant le beurre de karité ; une fois le mélange homogène, y ajouter 20 ml d'huile de noix de coco pure et 20 ml d'huile de monoï de telle sorte que le mélange forme une crème homogène et enfin y ajouter 55 ml d'huile d'olive vierge 1^{ère} pression pour former la crème.

3. Flacon doseur de 200 ml en plastique avec une pression doseur **caractérisé en ce qu'**il contient la crème selon l'une des revendications 1.

4. Lingette nettoyante épaisse carrée de 15 cm de côté **caractérisée en ce qu'**elle est imprégnée de la crème selon l'une des revendications 1.

5. Utilisation de la crème selon l'une des revendications 1 .pour supprimer toutes les tâches de peinture à l'huile, de peintures laquées, les tâches de feutres, stylos et cambouis sur la peau.

## Revendications modifiées

### Revendications modifiées conformément à la règle 137(2) CBE.

1. Utilisation d'une Crème pour supprimer toutes les tâches de peinture à l'huile, de peintures laquées, les tâches de feutres, stylos et cambouis sur la peau sans agresser la peau, sans risques d'allergies, non toxiques et non nocifs pour la santé en laissant la peau hydratée ; constituée exclusivement d'une combinaison de plusieurs agents hydratants et d'huiles naturelles:
• Huile de noix de coco pure : 20 ml
• Huile d'olive vierge 1^{ère} pression : 55 ml
• Huile d'amande douce : 20 ml
• Beurre de karité jaune pur non traité : 50 grammes
• Huile de monoï : 20 ml
La formation du complexe hydratant, de l'ensemble du mélange obtenu à l'aide des éléments constitutifs de la préparation a un effet nettoyant et détachant exclusivement obtenu avec les doses citées ci-dessus : en ce qu'elle est constituée d'une combinaison de plusieurs agents hydratants et d'huiles naturelles formant un complexe actif nettoyant et hydratant dont l'actif principal est l'huile de monoï : en effet, en plus de son action hydrante sur la peau: l'huile de monoï a un pouvoir nettoyant renforcé dans la combinaison avec les huiles présentes dans la crème ; en l'absence de la combinaison de ces éléments la crème n'a pas son pouvoir nettoyant et elle perd donc son activité inventive.

2. Utilisation d'une crème selon la revendication 1 dans laquelle la crème est dispensée à partir d' un procédé de préparation de la crème **caractérisé en ce qu'**il consiste à mettre dans un récipient 50 grammes de beurre de karité jaune pur non traité, ensuite incorporer 20 ml d'huile d'amande douce et mélanger dans le sens des aiguilles d'une montre en écrasant le beurre de karité ; une fois le mélange homogène, y ajouter 20 ml d'huile de noix de coco pure et 20 ml d'huile de monoï de telle sorte que le mélange forme une crème homogène et enfin y ajouter 55 ml d'huile d'olive vierge 1^{ère} pression pour former la crème. Selon la formation du mélange, l'invention en plus de son actif hydratant, la crème constituée a un pouvoir innovant : un pouvoir détachant et ce obtenu grâce aux quantités présentes au sein du procédé de préparation et de la quantité introduite dans les éléments constitutifs de la crème.

3. Utilisation d'une crème selon les revendication 1 et 2 dispensée à partir d'un Flacon doseur de 200 ml en plastique avec une pression doseur **caractérisé en ce qu'**il contient la crème.

4. Utilisation d'une crème selon les revendications 1 et 2 dispensée à partir d'une lingette nettoyante épaisse carrée de 15 cm de côté **caractérisée en ce qu'**elle est imprégnée de la crème.
